# EUROPÄISCHE PATENTANMELDUNG

(11) **EP 2 123 279 A1**
(43) Veröffentlichungstag der Anmeldung: **25.11.2009**
(21) Anmeldenummer: 08075496.3
(22) Anmeldetag: 14.05.2008
(51) Int. Cl.: A61K 31/567, A61P 5/34, A61P 5/36

(54) **Sequentielle Verabreichung von 20,20,21,21,21-Pentafluor-17-hydroxy-1 1beta-[4-(hydroxyacetyl) phenyl]-19-nor-17alpha-pregna-4,9-dien-3-on und einem oder mehreren Gestagenen zur Behandlung gynäkologischer Erkrankungen**

(71) Anmelder: Bayer Schering Pharma Aktiengesellschaft, 13353 Berlin (DE)
(72) Erfinder: Möller, Carsten, 10115 Berlin (DE); Fuhrmann, Ulrike, 10587 Berlin (DE); Schwede, Wolfgang, 16548 Glienicke (DE)

(57) **Zusammenfassung**

Die vorliegende Erfindung betrifft Behandlungsregime und Kombinationspräparate von 20,20,21,21,21-Pentafluor-17-hydroxy-11β-[4-(hydroxyacetyl)phenyl]-19-nor-17a-pregna-4,9-dien-3-on der Formel

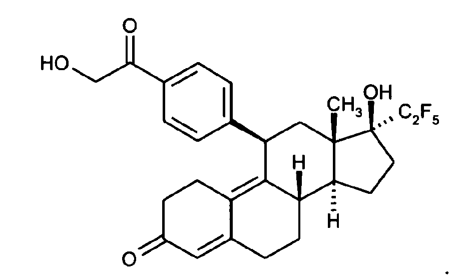

Insbesondere betrifft diese Erfindung sequentielle Regime zur Behandlung gynäkologischer Erkrankungen bei denen in einer ersten Phase der oben genannte Progesteronantagonist und in einer zweiten Phase ein Gestagen verabreicht wird.

## Beschreibung

Die vorliegende Erfindung betrifft Behandlungsregime und Kombinationspräparate von 20,20,21,21,21-Pentafluor-17-hydroxy-11β-[4-(hydroxyacetyl)phenyl]-19-nor-17α-pregna-4,9-dien-3-on der Formel

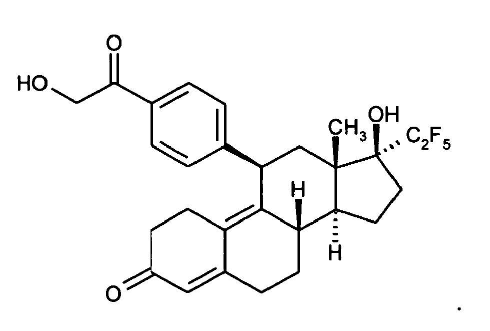

Insbesondere betrifft diese Erfindung sequentielle Regime zur Behandlung gynäkologischer Erkrankungen bei denen in einer ersten Phase der oben genannte Progesteronantagonist und in einer zweiten Phase ein Gestagen verabreicht wird.

Die Erfindung betrifft ebenfalls Behandlungsregime in denen 11β-(4-Acetylphenyl)-20,20,21,21,21-pentafluor-17-hydroxy-19-nor-17α-pregna-4,9-dien-3-on (Lonaprisan) verwendet wird.

Verbindungen mit antigestagener Wirksamkeit (kompetitive Progesteronrezeptorantagonisten) sind erstmals 1982 bekannt geworden (RU 486; EP57115) und seither zahlreich beschrieben worden. Vom oben genannten Progesteronrezeptorantagonisten strukturell verschiedene antigestagen wirksame Steroide mit fluorierter 17α-Seitenkette - unter anderem auch 11β-(4-Acetylphenyl)-20,20,21,21,21-pentafluor-17-hydroxy-19-nor-17α-pregna-4,9-dien-3-on - wurden in WO98/34947 und Fuhrmann et al., J. Med. Chem. 43, 5010 - 5016 (2000) veröffentlicht.

20,20,21,21,21-Pentafluor-17-hydroxy-11β-[4-(hydroxyacetyl)phenyl]-19-nor-17α-pregna-4,9-dien-3-on kann wie folgt hergestellt werden:

### i) 11β-(4-Ethenylphenyl)-5-hydroxy-5α-estr-9-en-3,17-dion-3-(2,2-dimethylpropan-1,3-diyl)ketal

3,3 g Magnesiumspäne werden unter Schutzgas in 14 ml absolutem Tetrahydrofuran vorgelegt und mit einem Tropfen 1,2-Dibromethan versetzt. Nach Eintreten der Reaktion wird eine Lösung von 25 g 4-Bromstyrol in 137 ml absolutem Tetrahydrofuran langsam so zugetropft, dass die Innentemperatur im Bereich von 40 bis 45°C bleibt. Das Reaktionsgemisch wird eine Stunde nachgerührt, bis das Magnesium vollständig umgesetzt ist. Dann wird der Ansatz mit 2,26 g Kupfer(I)chlorid versetzt. Eine Lösung von 8,5 g 5,10-Epoxy-5a,10a-estr-9(11)-en-3,17-dion-3-(2,2-dimethylpropan-1,3-diyl)ketal (Herstellung siehe Tetrahedron Lett. 26, 2069-2072 (1985)) in 137 ml absolutem Tetrahydrofuran wird langsam zugetropft. Das Reaktionsgemisch wird eine Stunde bei Raumtemperatur gerührt und dann auf gesättigte wässrige Ammoniumchloridlösung gegossen. Man extrahiert die wässrige Phase mit Ethylacetat, vereinigt die organischen Phasen, wäscht sie mit gesättigter wässriger Natriumchloridlösung und trocknet sie über Natriumsulfat. Man filtriert und engt im Vakuum ein. Säulenchromatographie an Kieselgel mit einem Gemisch aus Hexan/Ethylacetat ergibt 6,76 g der Titelverbindung als farblosen Schaum.
¹H-NMR (300 MHz, CDCl₃): δ= 7,30 d (*J*=9 Hz, 2H, aryl); 7,18 d (*J*=9 Hz, 2H, aryl); 6,66 dd (*J*=17 Hz + 11 Hz, 1H, vinyl); 5,70 dbr (*J*=17 Hz, 1H, vinyl); 5,20 dbr (*J*=11 Hz, 1H, vinyl); 4,44 s (1H, 5-OH); 4,29 dbr (*J*=6,5 Hz, 1H, H-11); 3,53 m (2H, 3-ketal); 3,51 m (*J*=11,4 Hz, 1H, 3-ketal); 3,42 m (*J*=11,4 Hz, 1H, 3-ketal); 1,05 s (3H, 3-ketal); 0,85 s (3H, 3-ketal); 0,49 s (3H, H-18).

### ii) 11β-(4-Ethenylphenyl)-20,20,21,21,21-pentafluor-5,17-dihydroxy-19-nor-5α,17α-pregn-9-en-3-on-(2,2-dimethylpropan-1,3-diyl)ketal

27,9 g kondensiertes Pentafluoriodethan in 140 ml absolutem Toluol wird mit einer Lösung von 6,76 g der unter i) hergestellten Verbindung in 140 ml absolutem Toluol bei -78°C versetzt. Man addiert bei dieser Temperatur 66,3 ml einer 1,5 molaren Lösung von Methyllithium-Lithiumbromid-Komplex in Diethylether. Anschließend wird eine Stunde bei 0°C nachgerührt. Dann wird das Reaktionsgemisch auf gesättigte wässrige Ammoniumchloridlösung gegossen. Man extrahiert mit Ethylacetat, wäscht mit gesättigter wässriger Natriumchloridlösung, trocknet über Natriumsulfat und engt im Vakuum ein. Chromatographie des erhaltenen Rohprodukts an Kieselgel mit einem Gemisch aus Hexan/Ethylacetat ergibt 3,73 mg der Titelverbindung als weißen Schaum.
¹H-NMR (300 MHz, CDCl₃): δ= 7,30 d (*J*=9 Hz, 2H, aryl); 7,17 d (*J*=9 Hz, 2H, aryl); 6,67 dd (*J*=17 Hz + 11 Hz, 1H, vinyl); 5,71 dbr (*J*=17 Hz, 1H, vinyl); 5,20 dbr (*J*=11 Hz, 1H, vinyl); 4,45 s (1H, 5-OH); 4,31 dbr (*J*=6,5 Hz, 1H, H-11); 3,53 m (2H, 3-ketal); 3,51 m (*J*=11,4 Hz, 1H, 3-ketal); 3,42 m (*J*=11,4 Hz, 1H, 3-ketal); 1,05 s (3H, 3-ketal); 0,85 s (3H, 3-ketal); 0,54 s (3H, H-18).

### iii) 11β-[4-(1,2-Dihydroxyethyl)phenyl]-20,20,21,21,21-pentafluor-5,17-dihydroxy-19-nor-5α,17α-pregn-9-en-3-on-(2,2-dimethylpropan-1,3-diyl)ketal

Zu einer Lösung von 1 g der nach ii) hergestellten Verbindung in 8,4 ml Tetrahydrofuran werden 1,68 ml einer wässrigen pH 7,00-Pufferlösung von Kaliumdihydrogenphosphat und Dikaliumhydrogenphosphat sowie 206 mg Trimethylamin-N-oxid gegeben. Bei 0°C werden 4,3 ml einer Lösung von 250 mg Osmiumtetroxid in 50 ml Butanol hinzugetropft. Das Reaktionsgemisch wird drei Stunden bei Raumtemperatur gerührt und dann auf gesättigte wässrige Natriumthiosulfatlösung gegossen. Man extrahiert mit Ethylacetat, wäscht mit gesättigter wässriger Natriumchloridlösung, trocknet über Natriumsulfat und engt im Vakuum ein. Chromatographie des erhaltenen Rohprodukts an Kieselgel mit einem Gemisch aus Hexan/Ethylacetat ergibt 860 mg der Titelverbindung als weißen Schaum. Man erhält ein Epimerengemisch am Benzylcarbinol.
¹H-NMR (300 MHz, CDCl₃): δ= 7,25 d (*J*=9 Hz, 2H, aryl); 7,20 d (*J*=9 Hz, 2H, aryl); 4,78 m (1H, CHOH); 4,44 s (1H, 5-OH); 4,32 dbr (*J*=6,5 Hz, 1H, H-11); 3,73 m (1H, CH₂OH); 3,65 m (1H, CH₂OH); 3,54 m (2H, 3-ketal); 3,52 m (*J*=11,0 Hz, 1H, 3-ketal); 3,44 m (*J*=11,0 Hz, 1H, 3-ketal); 1,04 s (3H, 3-ketal); 0,87 s (3H, 3-ketal); 0,51 s (3H, H-18).

### iv) 11β-[4-(1,2-Dihydroxyethyl)phenyl]-20,20,21,21,21-pentafluor-17-hydroxy-19-nor-17α-pregna-4,9-dien-3-on

200 mg der unter iii) beschriebenen Verbindung werden in 3 ml Methanol mit 141 µl wässriger halbkonzentrierter Schwefelsäure eine Stunde bei Raumtemperatur gerührt. Anschließend wird auf gesättigte wässrige Natriumhydrogencarbonatlösung gegossen und mit Ethylacetat extrahiert. Man wäscht die organische Phase mit gesättigter wässriger Natriumchloridlösung, trocknet über Natriumsulfat, filtriert und engt im Vakuum ein. Säulenchromatographie an Kieselgel mit einem Gemisch aus Hexan/Ethylacetat ergibt 78 mg der Titelverbindung als farblosen Schaum. Man erhält ein Epimerengemisch am Benzylcarbinol.
¹H-NMR (300 MHz, CDCl₃): δ= 7,25 d (*J*=9 Hz, 2H, aryl); 7,15 d (*J*=9 Hz, 2H, aryl); 5,77 s (1H, H-4); 4,74 m (1H, CHOH); 4,42 dbr (*J*=7 Hz, 1H, H-11); 3,69 m (1H, CH₂OH); 3,59 m (1H, CH₂OH); 0,56 s (3H, H-18).

### v) 20,20,21,21,21-Pentafluor-11β-[4-(hydroxyacetyl)phenyl]-5,17-dihydroxy-19-nor-5α,17α-pregn-9-en-3-on-(2,2-dimethylpropan-1,3-diyl)ketal

Zu 3,6 mg Chromtrioxid in 7 ml Dichlormethan werden bei Raumtemperatur 283 µl *tert*-Butylhydroperoxid getropft. Anschließend wird eine Lösung von 450 mg der unter iii) hergestellten Verbindung in 7 ml Dichlormethan hinzugetropft. Das Gemisch wird drei Stunden bei Raumtemperatur gerührt. Die Reaktion wird durch Zugabe von Dimethylsulfid abgebrochen. Man wäscht mit gesättigter wässriger Natriumchloridlösung, trocknet über Natriumsulfat und engt im Vakuum ein. Chromatographie des erhaltenen Rohprodukts an Kieselgel mit einem Gemisch aus Hexan/Ethylacetat ergibt 87 mg der Titelverbindung als weißen Schaum.
¹H-NMR (300 MHz, CDCl₃): δ= 7,83 d (*J*=9 Hz, 2H, aryl); 7,37 d (*J*=9 Hz, 2H, aryl); 4,84 m (2H, CH₂OH); 4,37 dbr (*J*=6,5 Hz, 1H, H-11); 3,53 m (2H, 3-ketal); 3,47 m (*J*=11,0 Hz, 1H, 3-ketal); 3,42 m (*J*=11,0 Hz, 1H, 3-ketal); 1,04 s (3H, 3-ketal); 0,85 s (3H, 3-ketal); 0,49 s (3H, H-18).

### vi) 20,20,21,21,21-Pentafluor-17-hydroxy-11β-[4-(hydroxyacetyl)phenyl]-19-nor-17a-pregna-4,9-dien-3-on

87 mg der unter v) beschriebenen Verbindung werden analog zu dem unter iv) beschriebenen Verfahren in 1,4 ml Methanol mit 62 µl wässriger halbkonzentrierter Schwefelsäure zu 25 mg der Titelverbindung als farblosem Schaum umgesetzt.
¹H-NMR (300 MHz, CDCl₃): δ**=** 7,86 d (*J*=9 Hz, 2H, aryl); 7,34 d (*J*=9 Hz, 2H, aryl); 5,81 s (1H, H-4); 4,85 dbr (*J*=5 Hz, 2H, CH₂OH); 4,50 dbr (*J*=7 Hz, 1H, H-11); 3,50 tbr (*J*=5 Hz, 1H, OH); 0,57 s (3H, H-18).

Die Beispiele 3a und 3b von US 11/984,331 bzw. PCT/EP2007/009997 in denen weitere Herstellungsverfahren von 20,20,21,21,21-Pentafluor-17-hydroxy-11β-[4-(hydroxyacetyl)phenyl]-19-nor-17α-pregna-4,9-dien-3-on offenbart sind, werden hiermit zum Bestandteil der vorliegenden Anmeldung gemacht.

Die Applikation von Substanzen mit antagonistischer Eigenschaft am Progesteron Rezeptor (PR-Antagonisten und SPRMs bzw. selektive Progesteron-Receptor-Modulatoren) führt - wenn sie über einen längeren Zeitraum erfolgt - zu Veränderungen der Endometriums-Morphologie. Diese Veränderungen sind von Bedeutung für alle Langzeitanwendungen, sei es zur Behandlung gynäkologischer Erkrankungen wie der Endometriose, von uterinen Leiomyomen oder zur Behandlung von abnormalen bzw. dysfunktionalen Menstruationsblutungen. In präklinischen Experimenten konnte durch Untersuchungen an Primaten demonstriert werden, dass PR-antagonistische Verbindungen eine antiproliferative Wirkung an Endometriumszellen haben. Diese wird auch als nicht-kompetitiver anti-östrogener Effekt bezeichnet. Begleitend mit diesem antiproliferativen Effekt wurde eine Kompaktierung des endometrialen Stromas beobachtet sowie eine zystische Erweiterung der endometrialen Drüsen (HODGEN GD, VAN UEM JF, CHILLIK CF, DANFORTH DR, WOLF JP, NEULEN J, WILLIAMS RF, CHWALISZ K: Noncompetitive anti-oestrogenic activity of progesterone antagonists in primate models. Hum Reprod (1994) 9 Suppl 1, 77-81; SLAYDEN OD, ZELINSKI-WOOTEN MB, CHWALISZ K, STOUFFER RL, BRENNER RM: Chronic treatment of cycling rhesus monkeys with low doses of the antiprogestin ZK 137 316: morphometric assessment of the uterus and oviduct. Hum Reprod (1998) 13(2), 269-77; SLAYDEN OD, BRENNER RM: RU 486 action after estrogen priming in the endometrium and oviducts of rhesus monkeys (Macaca mulatta). J Clin Endocrinol Metab (1994) 78(2), 440-8). Die Anwendung des PR-Antagonisten Mifepriston in Patientinnen führte zu ähnlichen Effekten: zystische Erweiterung der Endometriumsdrüsen, ein inaktives Endometriumsepithel sowie eine reduzierte mitotische Aktivität des Endometriums (NARVEKAR N, CAMERON S, CRITCHLEY HO, LIN S, CHENG L, BAIRD DT: Low-dose mifepristone inhibits endometrial proliferation and up-regulates androgen receptor. J Clin Endocrinol Metab (2004) 89(5), 2491-7; BAIRD DT, BROWN A, CRITCHLEY HO, WILLIAMS AR, LIN S, CHENG L: Effect of long-term treatment with low-dose mifepristone on the endometrium. Hum Reprod (2003) 18(1), 61-8). Ausserdem ist bekannt, dass die Anwendung von PR-Antagonisten und SPRMs über einen längeren - also die Dauer eines Menstruationszyklus übersteigenden - Zeitraum zu einem Ausbleiben der Regelblutungen führt. Sie induzieren eine Amenorrhoe (NARVEKAR N, CRITCHLEY HO, CHENG L, BAIRD DT: Mifepristoneinduced amenorrhoea is associated with an increase in microvessel density and glucocorticoid receptor and a decrease in stromal vascular endothelial growth factor. Hum Reprod (2006) 21(9), 2312-8). Die histologischen Veränderungen, die durch die Applikationen von PR-Antagonisten und SPRMs am humanen Endometrium induziert werden, lassen sich nicht mit den gängigen pathologischen Kategorien beschreiben (HORNE FM, BLITHE DL: Progesterone receptor modulators and the endometrium: changes and consequences. Hum Reprod Update (2007) 13(6): 567-80. Die Evaluierung solcher Endometriumsbiopsien wird durch diese einzigartige Morphologie sowie durch eine fehlende Klassifizierung erschwert. So wurde z.B. bei Patientinnen unter Mifepriston-Behandlung eine gesteigerte Endometriumsdicke mittels eines transvaginalen Ultraschalls diagnostiziert. Möglicherweise wurde diese durch die oben beschriebenen glanduläre Dilatation hervorgerufen. Andere Studien beschreiben eine Endometriums-Hyperplasie ohne Atypie in Patientinnen nach Langzeitbehandlung mit Mifepriston (EISINGER SH, MELDRUM S, FISCELLA K, et al. Low-dose mifepristone for uterine leiomyomata. Obstet Gynecol 2003 ; 101 : 243 - 50). Diese Beobachtungen werden aber kontrovers beurteilt, eine Re-Evaluierung dieser Patientinnen-Proben führte zu einer Deklassizifizierung einiger Patientinnen (Eisinger SH, Bonfi glio T, Fiscella K, et al. Twelve-month safety and effi cacy of low-dose mifepristone for uterine myomas. J Minim Invasive Gynecol 2005 ; 12 : 227 - 33). In einer kürzliche veröffentlichten Studie zur Wirkung des SPRM Asoprisnil wurde nach 3-monatiger Anwendung im Endometrium ein Muster eines "nichtphysiologischen sekretorischen Effektes" diagonistiziert: Das Endometrium zeigte ein niedriges Niveau stromaler und glandulärer Proliferation, entwickelte teilweise sekretorische glanduläre Eigenschaften und stromale Veränderungen. Zusätzlich wurde eine Verdickung der Glattmuskelzellschicht beschrieben, die die Endometriumsgefäße umschließen (WILLIAMS AR, CRITCHLEY HO, OSEI J, INGAMELLS S, CAMERON IT, HAN C, CHWALISZ K: The effects of the selective progesterone receptor modulator asoprisnil on the morphology of uterine tissues after 3 months treatment in patients with symptomatic uterine leiomyomata. Hum Reprod (2007) 22(6), 1696-704).

Für eine sichere, wirksame Langzeitanwendung von PR-Antagonisten in Patientinnen sollte eine Reversibilität der beschriebenen Endometriumseffekte gewährleistet sein, zum einen, um ein Sicherheitsrisiko für die Patientinnen auszuschließen, zum anderen, um einer gesteigerten Gefahr einer Fehldiagnose entgegenzuwirken.

Sequentielle Behandlungsregime kompetitiver Progesteronantagonisten mit Gestagenen sind z. B. in US 6,043,234, von SPRMs mit Gestagenen in US 2005/0215535 und US 2005/0215536 offenbart.

Aufgabe der vorliegenden Erfindung ist es, ein Arzneimittel bereit zu stellen, welches für die sichere Langzeitbehandlung von gynäkologischen Erkrankungen wie Endometriose, uterinen Leiomyomen oder dysfunktionalen Menstruationsblutungen geeignet ist. Dieses Arzneimittel soll neben der Effektivität in den genannten Indikationen sowohl eine Reversibilität der beschriebenen Effekte am Endometrium und damit eine erhöhte Sicherheit des Uterus gewährleisten, eine kontrollierte Menstruation ermöglichen und mögliche Fehldiagnosen durch die potentiell induzierte Endometriumsdicke verhindern.

Die vorliegende Erfindung löst diese Aufgabe durch den diskontinuierlichen Einsatz des Progesteronrezeptorantagonisten 20,20,21,21,21-Pentafluor-17-hydroxy-11β-[4-(hydroxyacetyl)phenyl]-19-nor-17α-pregna-4,9-dien-3-on.

Dazu wird der Progesteronrezeptorantagonist 20,20,21,21,21-Pentafluor-17-hydroxy-11β-[4-(hydroxyacetyl)phenyl]-19-nor-17α-pregna-4,9-dien-3-on in einem Behandlungszyklus oder mehreren, unmittelbar aufeinander folgenden Behandlungszyklen verabreicht, wobei jeder Behandlungszyklus aus zwei Behandlungszeiträumen besteht.

Im ersten Behandlungszeitraum eines jeden Behandlungszyklus wird über 28 - 168, bevorzugt 56 - 112, besonders bevorzugt 84 Tagen der Progesteronrezeptorantagonist 20,20,21,21,21-Pentafluor-17-hydroxy-11β-[4-(hydroxyacetyl)phenyl]-19-nor-17α-pregna-4,9-dien-3-on gegeben.

Nach Ende dieses ersten Behandlungszeitraums folgt in jedem Behandlungszyklus ein zweiter Behandlungszeitraum in dem kein Progesteronrezeptorantagonist gegeben wird.

In diesem zweiten Behandlungszeitraum eines jeden Behandlungszyklus werden entweder gestagenhaltige (Gestagenmonopräparate oder Gestagenkombinationspräparate zum Beispiel mit Estrogenen, wie zum Beispiel handelsübliche Kontrazeptiva oder Präparate zur Hormonersatztherapie) oder gestagenfreie Dosiseinheiten enthaltend andere Wirkstoffe oder gar keine Dosiseinheiten verabreicht.

Werden im zweiten Behandlungszeitraum gestagenhaltige Dosiseinheiten verabreicht, so beträgt die Dauer dieses zweiten Behandlungszeitraums 1 bis 30, bevorzugt 5 bis 20, besonders bevorzugt 7 bis 14 Tage. Insbesondere sind zweite Behandlungszeiträume mit einer Dauer von 7, 10, 11, 12, 14, 21 oder 28 Tagen denkbar.

Als Gestagene kommen Substanzen in Betracht, die kontrazeptiv wirken und in der Lage sind Entzugsblutungen zu induzieren. Dazu gehören z. B. Chlormanidonacetat, Cyproteronacetat, Desogestrel, Dienogest, Drospirenon, Dydrogesterone, Ethynodioldiacetat, Etonorgestrel, Gestoden, Levonorgestrel, Medrogeston, Medroxyprogesteron und Medroxyprogesteronacetat, Norethindron, Norethisteron und Norethisteronacetat, Norgestimat, Norgestrel, Progesteron, Promegeston oder Trimegeston.

Ergänzend aber nicht notwendig wäre die Gabe eines Estrogens, z. B. von Ethinylestradiol, von Estradiol, Estriol, Estetrol, Estron oder konjugierten equinen Estrogenen denkbar. Die entsprechenden Gestagen- bzw. Estrogendosierungen können teilweise der Patentanmeldung US 2005/0215536, Absatz 13 - 15 entnommen werden, der hiermit zum Bestandteil der vorliegenden Anmeldung gemacht wird. Weitere Dosierungsvorschläge finden sich zum Beispiel in der Roten Liste von 2007.

Unmittelbar nachfolgend würde dann der nächste Behandlungzyklus mit dem Progesteronrezeptorantagonisten - wie oben beschrieben - beginnen. Durch die direkte Applikation des Progesteronrezeptorantagonisten im Anschluss an die Gabe des Gestagens wird erwartungsgemäß eine Menstruation induziert.

Werden im zweiten Behandlungszeitraum gestagenfreie Dosiseinheiten enthaltend andere Wirkstoffe oder gar keine Dosiseinheiten verabreicht, so sollte mit dem nächsten Behandlungszyklus - also der erneuten Gabe des Progesteronrezeptorantagonisten im ersten Behandlungszeitraum des folgenden Behandlungszyklus - bis zum Eintreten der Regelblutung gewartet werden (flexible Pause). Erwartungsgemäß sollte diese bei den meisten Frauen innerhalb von 30 Tage nach dem Absetzen des Progesteronrezeptorantagonisten erfolgen. Im Einzelfall kann diese menstruationsfreie Periode aber auch länger dauern. Bevorzugt ist hier ein Zeitraum von 2 - 4 Wochen.

Ebenfalls denkbar wäre es, den zweiten Behandlungszeitraum eines jeden Behandlungszyklus nochmals in zwei weitere Behandlungsperioden zu unterteilen, wobei in der ersten Behandlungsperiode die gestagenhaltigen, gegebenenfalls auch ergänzend estrogenhaltigen Dosiseinheiten, in der sich daran unmittelbar anschließenden zweiten Behandlungsperiode die gestagenfreien Dosiseinheiten enthaltend andere Wirkstoffe oder gar keine Dosiseinheiten verabreicht werden. Auch in diesem Falle sollte mit dem nächsten Behandlungszyklus - also der erneuten Gabe des Progesteronrezeptorantagonisten im ersten Behandlungszeitraum des folgenden Behandlungszyklus - bis zum Eintreten der Regelblutung gewartet werden. Erwartungsgemäß erfolgt dies nach dem Absetzen des Gestagens. Ebenfalls möglich erscheint der Beginn des nächsten Behandlungszyklus nach Ende der Menstruation. Je nach Anzahl der zuvor verabreichten gestagenhaltigen täglichen Dosiseinheiten (z.B. 7, 10, 11, 12, 14, 21) wäre auch die Gabe von n gestagenfreien Dosiseinheiten denkbar, wobei n = 28 - "Anzahl der zuvor verabreichten gestagenhaltigen täglichen Dosiseinheiten" ist.

Zusätzlich können über die gesamte Behandlungsdauer oder einzelne Teile davon, also während des gesamten Behandlungszyklus, insbesondere während des ersten und/oder zweiten Behandlungszeitraums und/oder während der ersten und/oder zweiten Behandlungsperiode des zweiten Behandlungszeitraums weitere Wirkstoffe - wie z.B. Folsäure und deren Salze oder Metafolin - verabreicht werden. Zur Illustration der Begriffe Behandlungszyklus, Behandlungszeitraum und Behandlungsperiode vergl. die folgende Tabelle:

| Behandlungszyklus | | |
|---|---|---|
| | | |

| erster Behandlungszeitraum | zweiter Behandlungszeitraum | |
|---|---|---|
| | | |

| | erste Behandlungsperiode | zweite Behandlungsperiode |
|---|---|---|
| | | |
| Gabe des Progesteronrezeptorantagonisten | Gabe des Gestagens | fehlt |
| | | |
| Gabe des Progesteronrezeptorantagonisten | Gabe des Gestagens | flexible Pause |
| | | |
| Gabe des Progesteronrezeptorantagonisten | flexible Pause | fehlt |

Fehlt die zweite Behandlungsperiode - besteht also der zweite Behandlungszeitraum nur aus einer, nämlich der ersten Behandlungsperiode - so folgt der nächste Behandlungszyklus unmittelbar nach Beendigung der Gestagengabe oder der flexiblen Pause.

Anstelle der Gabe von 20,20,21,21,21-Pentafluor-17-hydroxy-11β-[4-(hydroxyacetyl)phenyl]-19-nor-17α-pregna-4,9-dien-3-on ist die Gaben von 11β-(4-Acetylphenyl)-20,20,21,21,21-pentafluor-17-hydroxy-19-nor-17α-pregna-4,9-dien-3-on (Lonaprisan) mit im wesentlichen gleichem klinischem Erfolg denkbar.

Insbesondere sind folgende Behandlungsregime denkbar:

| **Behandlungszyklus** | | | **anschließend** |
|---|---|---|---|
| **erster Behandlungszeitraum** | **zweiter Behandlungszeitraum** | | |
| | 1. Behandlungsperiode | 2. Behandlungsperiode | |
| 56 Tage Progesteronrezeptorantagonist | | Flexible Pause | ggf. nächster Behandlungszyklus |
| 56 Tage Progesteronrezeptorantagonist | 7 Tage Gestagen | Flexible Pause | ggf. nächster Behandlungszyklus |
| 56 Tage Progesteronrezeptorantagonist | 14 Tage Gestagen | Flexible Pause | ggf. nächster Behandlungszyklus |
| 56 Tage Progesteronrezeptorantagonist | 21 Tage Gestagen | Flexible Pause | ggf. nächster Behandlungszyklus |
| 56 Tage Progesteronrezeptorantagonist | 28 Tage Gestagen | Flexible Pause | ggf. nächster Behandlungszyklus |
| 56 Tage Progesteronrezeptorantagonist | 28 Tage Gestagen/Estrogen | Flexible Pause | ggf. nächster Behandlungszyklus |
| | | | |
| 84 Tage Progesteronrezeptorantagonist | | Flexible Pause | ggf. nächster Behandlungszyklus |
| 84 Tage Progesteronrezeptorantagonist | 7 Tage Gestagen | Flexible Pause | ggf. nächster Behandlungszyklus |
| 84 Tage Progesteronrezeptorantagonist | 14 Tage Gestagen | Flexible Pause | ggf. nächster Behandlungszyklus |
| 84 Tage Progesteronrezeptorantagonist | 21 Tage Gestagen | Flexible Pause | ggf. nächster Behandlungszyklus |
| 84 Tage Progesteronrezeptorantagonist | 28 Tage Gestagen | Flexible Pause | ggf. nächster Behandlungszyklus |
| 84 Tage Progesteronrezeptorantagonist | 28 Tage Gestagen/Estrogen | Flexible Pause | ggf. nächster Behandlungszyklus |
| | | | |
| 112 Tage Progesteronrezeptorantagonist | | Flexible Pause | ggf. nächster Behandlungszyklus |
| 112 Tage Progesteronrezeptorantagonist | 7 Tage Gestagen | Flexible Pause | ggf. nächster Behandlungszyklus |
| 112 Tage Progesteronrezeptorantagonist | 14 Tage Gestagen | Flexible Pause | ggf. nächster Behandlungszyklus |
| 112 Tage Progesteronrezeptorantagonist | 21 Tage Gestagen | Flexible Pause | ggf. nächster Behandlungszyklus |
| 112 Tage Progesteronrezeptorantagonist | 28 Tage Gestagen | Flexible Pause | ggf. nächster Behandlungszyklus |
| 112 Tage Progesteronrezeptorantagonist | 28 Tage Gestagen/Estrogen | Flexible Pause | ggf. nächster Behandlungszyklus |
| | | | |
| 56 Tage Progesteronrezeptorantagonist | 7 Tage Gestagen | Fehlt | ggf. nächster Behandlungszyklus |
| 56 Tage Progesteronrezeptorantagonist | 14 Tage Gestagen | Fehlt | ggf. nächster Behandlungszyklus |
| 56 Tage Progesteronrezeptorantagonist | 21 Tage Gestagen | Fehlt | ggf. nächster Behandlungszyklus |
| 56 Tage Progesteronrezeptorantagonist | 28 Tage Gestagen | Fehlt | ggf. nächster Behandlungszyklus |
| 56 Tage Progesteronrezeptorantagonist | 28 Tage Gestagen/Estrogen | Fehlt | ggf. nächster Behandlungszyklus |
| | | | |
| 84 Tage Progesteronrezeptorantagonist | 7 Tage Gestagen | Fehlt | ggf. nächster Behandlungszyklus |
| 84 Tage Progesteronrezeptorantagonist | 14 Tage Gestagen | Fehlt | ggf. nächster Behandlungszyklus |
| 84 Tage Progesteronrezeptorantagonist | 21 Tage Gestagen | Fehlt | ggf. nächster Behandlungszyklus |
| 84 Tage Progesteronrezeptorantagonist | 28 Tage Gestagen | Fehlt | ggf. nächster Behandlungszyklus |
| 84 Tage Progesteronrezeptorantagonist | 28 Tage Gestagen/Estrogen | Fehlt | ggf. nächster Behandlungszyklus |
| | | | |
| 112 Tage Progesteronrezeptorantagonist | 7 Tage Gestagen | Fehlt | ggf. nächster Behandlungszyklus |
| 112 Tage Progesteronrezeptorantagonist | 14 Tage Gestagen | Fehlt | ggf. nächster Behandlungszyklus |
| 112 Tage Progesteronrezeptorantagonist | 21 Tage Gestagen | Fehlt | ggf. nächster Behandlungszyklus |
| 112 Tage Progesteronrezeptorantagonist | 28 Tage Gestagen | Fehlt | ggf. nächster Behandlungszyklus |
| 112 Tage Progesteronrezeptorantagonist | 28 Tage Gestagen/Estrogen | Fehlt | ggf. nächster Behandlungszyklus |
| | | | |
| 56 Tage Progesteronrezeptorantagonist | 7 Tage Gestagen | 21 Tage Placebo | ggf. nächster Behandlungszyklus |
| 56 Tage Progesteronrezeptorantagonist | 14 Tage Gestagen | 14 Tage Placebo | ggf. nächster Behandlungszyklus |
| 56 Tage Progesteronrezeptorantagonist | 21 Tage Gestagen | 7 Tage Placebo | ggf. nächster Behandlungszyklus |
| | | | |
| 84 Tage Progesteronrezeptorantagonist | 7 Tage Gestagen | 21 Tage Placebo | ggf. nächster Behandlungszyklus |
| 84 Tage Progesteronrezeptorantagonist | 14 Tage Gestagen | 14 Tage Placebo | ggf. nächster Behandlungszyklus |
| 84 Tage Progesteronrezeptorantagonist | 21 Tage Gestagen | 7 Tage Placebo | ggf. nächster Behandlungszyklus |
| | | | |
| 112 Tage Progesteronrezeptorantagonist | 7 Tage Gestagen | 21 Tage Placebo | ggf. nächster Behandlungszyklus |
| 112 Tage Progesteronrezeptorantagonist | 14 Tage Gestagen | 14 Tage Placebo | ggf. nächster Behandlungszyklus |
| 112 Tage Progesteronrezeptorantagonist | 21 Tage Gestagen | 7 Tage Placebo | ggf. nächster Behandlungszyklus |
| | | | |
| 56 Tage Progesteronrezeptorantagonist | 21 Tage Placebo | Fehlt | ggf. nächster Behandlungszyklus |
| 56 Tage Progesteronrezeptorantagonist | 28 Tage Placebo | Fehlt | ggf. nächster Behandlungszyklus |
| | | | |
| 84 Tage Progesteronrezeptorantagonist | 21 Tage Placebo | Fehlt | ggf. nächster Behandlungszyklus |
| 84 Tage Progesteronrezeptorantagonist | 28 Tage Placebo | Fehlt | ggf. nächster Behandlungszyklus |
| | | | |
| 112 Tage Progesteronrezeptorantagonist | 21 Tage Placebo | Fehlt | ggf. nächster Behandlungszyklus |
| 112 Tage Progesteronrezeptorantagonist | 28 Tage Placebo | Fehlt | ggf. nächster Behandlungszyklus |

Als Progesteronrezeptorantagonist kommt vorrangig 20,20,21,21,21-Pentafluor-17-hydroxy-11β-[4-(hydroxyacetyl)phenyl]-19-nor-17α-pregna-4,9-dien-3-on in Frage. Als hiermit zu kombinierende Gestagene kommen vorrangig Drospirenon, Dienogest oder Levonorgestrel in Frage. Als Gestagen/Estrogen-Kombination kommen vorrangig kommerziell - z.B. in oralen Kontrazeptiva - verfügbare Kombinationen in Betracht. Placebo bedeutet nicht nur eine wirkstofffreie Dosisform, sondern auch Dosisformen, die von Progesteronrezeptorantagonisten, Gestagenen oder Gestagen/Estrogen-Kombinationen verschiedene Wirkstoffe, insbesondere Folsäure, deren Salze oder Metafolin enthalten. Anstelle der explizit erwähnten Zeiträume der ersten Behandlungsperiode kommen zumindest für die Blöcke 1 bis 9 auch Zeiträume von 10, 11 und 12 Tagen in betracht. Aus Platzgründen wurde darauf verzichtet, jede einzelne Unterkombination spezieller Wirkstoffe in den oben offenbarten Regimen ausdrücklich zu nennen. Es sollen aber alle oben genannten Kombinationen in denen die Worte
- Progesteronrezeptorantagonist durch 20,20,21,21,21-Pentafluor-17-hydroxy-11β-[4-(hydroxyacetyl)phenyl]-19-nor-17α-pregna-4,9-dien-3-on und Gestagen durch Drospirenon;
- Progesteronrezeptorantagonist durch 20,20,21,21,21-Pentafluor-17-hydroxy-11β-[4-(hydroxyacetyl)phenyl]-19-nor-17α-pregna-4,9-dien-3-on und Gestagen durch Dienogest;
- Progesteronrezeptorantagonist durch 20,20,21,21,21-Pentafluor-17-hydroxy-11β-[4-(hydroxyacetyl)phenyl]-19-nor-17α-pregna-4,9-dien-3-on und Gestagen durch Levonorgestrel oder
- Progesteronrezeptorantagonist durch 20,20,21,21,21-Pentafluor-17-hydroxy-11β-[4-(hydroxyacetyl)phenyl]-19-nor-17α-pregna-4,9-dien-3-on und Gestagen/Estrogen durch Drospirenon/Ethinylestradiol
ersetzt werden.

Neben den oben erwähnten Behandlungsregimen (sequentielle Einnahme) kommt - insbesondere zur Behandlung der Endometriose - auch ein Kombinationspräparat enthaltend den Progesteronrezeptorantagonisten 20,20,21,21,21 -Pentafluor-17-hydroxy-11β-[4-(hydroxyacetyl)phenyl]-19-nor-17α-pregna-4,9-dien-3-on und formuliert als fixe Kombination (gleichzeitige Einnahme) in Betracht. Anstelle der Formulierung als fixe Kombination ist auch eine getrennte Formulierung des Progesteronrezeptorantagonisten 20,20,21,21,21-Pentafluor-17-hydroxy-11β-[4-(hydroxyacetyl)phenyl]-19-nor-17α-pregna-4,9-dien-3-on und des Gestagens denkbar, wenn diese zur gleichzeitigen Einnahme bestimmt sind - insbesondere, wenn diese gemeinsam verpackt werden.

Ebenso denkbar wären Regime, in denen das Wort Progesteronrezeptorantagonist durch 11β-(4-Acetylphenyl)-20,20,21,21,21-pentafluor-17-hydroxy-19-nor-17α-pregna-4,9-dien-3-on (Lonaprisan) ersetzt wird - einschließlich der oben genannten Ersetzungen der Worte Gestagen oder Gestagen/Estrogen.

Bevorzugt sind in allen oben genannten Behandlungsregimen orale Dosisformen zur einmal täglichen Einnahme. Diese können in einer Arzneimittelpackung zur sequentiellen Einnahme zusammengefasst und mit einem Beipackzettel, der über die richtige Einnahmereihenfolge informiert, zusammengefasst werden.

20,20,21,21,21-Pentafluor-17-hydroxy-11β-[4-(hydroxyacetyl)phenyl]-19-nor-17α-pregna-4,9-dien-3-on kann durch - dem Fachmann geläufige Verfahren unter Verwendung physiologisch und pharmakologisch akzeptabler Hilfs- und Zusatzstoffe formuliert werden. Das gleiche gilt für 11β-(4-Acetylphenyl)-20,20,21,21,21-pentafluor-17-hydroxy-19-nor-17α-pregna-4,9-dien-3-on (Lonaprisan). Formulierungen von Gestagenen und Gestagen/Estrogen-Kombinationen gehören ebenfalls zum Stand der Technik und können diesem entnommen werden.

Die folgenden Beispiele dienen der Erläuterung der Erfindung ohne diese in irgend einer Weise zu beschränken.

### Beispiel 1:

36 gesunde, tuben-ligierte Frauen im Alter von 24 bis 43 Jahren wurden einer randomisierten Studie zum Effekt von Lonaprisan unterzogen. Hierzu wurden die Frauen in drei Gruppen aufgeteilt: 7 Frauen erhielten Placebo, 14 Frauen 1 mg Lonaprisan und 15 Frauen 10 mg Lonaprisan über einen Zeitraum von 3 Monaten, einmal täglich. Die kontinuierliche Behandlung durch die tägliche Gabe von Lonaprisan führte zu einer Inhibition der Ovulation bei den Patientinnen sowie einem Blutungsmuster, welches einer Amenorrhoe oder einer stark reduzierten Blutung entsprach. Beide Effekte wurden nicht in der Placebo-Gruppe beobachtet. Nach Absetzen von Lonaprisan kam es zu einer spontanen Rückkehr der Menstruationsblutung. Des Weiteren wurden durch Endometriumsbiospien nach der ersten Menstruationblutung nach Beendigung der Behandlungsphase keine persistierenden Veränderungen am Endometrium in den Patientinnen festgestellt.

### Beispiel 2:

Frauen im reproduktiven Alter werden in einer randomisierten Studie sequenziell in zwei Behandlungszyklen mit 20,20,21,21,21-Pentafluor-17-hydroxy-11β-[4-(hydroxyacetyl)phenyl]-19-nor-17α-pregna-4,9-dien-3-on behandelt. Hierzu wird den Frauen 20,20,21,21,21-Pentafluor-17-hydroxy-11β-[4-(hydroxyacetyl)phenyl]-19-nor-17α-pregna-4,9-dien-3-on je 84 Tage mit einer täglichen Dosis zwischen ca. 1 mg und ca. 50 mg verabreicht, gefolgt von der Einnahme eines Gestagens über einen Zeitraum von ca. 10 bis 14 Tagen. Dieser gesamte Behandlungszyklus wird einmal wiederholt. Klinische Endpunkte sind die respektive Krankheitssymptomatik, sowie das Blutungsmuster der Frauen, die Ovulationsinhibition und die Endometriumsmorphology. Die sequentielle Behandlung der Frauen mit 20,20,21,21,21-Pentafluor-17-hydroxy-11β-[4-(hydroxyacetyl)phenyl]-19-nor-17α-pregna-4,9-dien-3-on und dem Gestagen führt zu einer Menstruationsblutung im Anschluss an die Gabe des Gestagens. Während der Behandlungsphase mit 20,20,21,21,21-Pentafluor-17-hydroxy-11β-[4-(hydroxyacetyl)phenyl]-19-nor-17α-pregna-4,9-dien-3-on zeigen die Frauen ein Blutungsmuster, welches einer Amenorrhoe oder einer stark reduzierten Blutung entspricht. Endometriumsbiopsien nach Abschluss eines jeden Behandlungzyklus zeigen keine persistierenden Veränderungen am Endometrium der Patientinnen.

## Patentansprüche

1. Pharmazeutisches Kombinationspräparat enthaltend
- einzelne Dosiseinheiten des Progesteronrezeptorantagonisten 20,20,21,21,21-Pentafluor-17-hydroxy-11β-[4-(hydroxyacetyl)phenyl]-19-nor-17α-pregna-4,9-dien-3-on der Formel 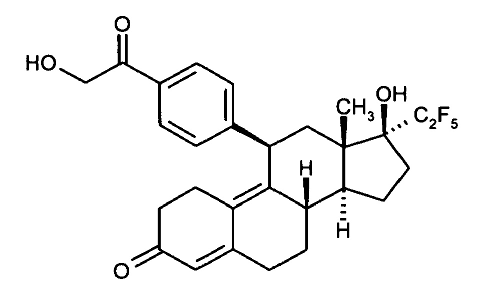 und
- einzelne Dosierungseinheiten eines Gestagens
zu dessen sequentieller, oraler Verabreichung zur Behandlung gynäkologischer Erkrankungen.

2. Kombinationspräparat gemäß Anspruch 1 enthaltend 28 bis 168 Dosiseinheiten des Progesteronrezeptorantagonisten.

3. Kombinationspräparat gemäß Anspruch 2 enthaltend 56 bis 112 Dosiseinheiten des Progesteronrezeptorantagonisten.

4. Kombinationspräparat gemäß Anspruch 3 enthaltend 84 Dosiseinheiten des Progesteronrezeptorantagonisten.

5. Kombinationspräparat gemäß Anspruch 1 enthaltend 1 bis 30 Dosiseinheiten des Gestagens.

6. Kombinationspräparat gemäß Anspruch 5 enthaltend 5 bis 20 Dosiseinheiten des Gestagens.

7. Kombinationspräparat gemäß Anspruch 6 enthaltend 7 bis 14 Dosiseinheiten des Gestagens.

8. Kombinationspräparat gemäß Anspruch 1 enthaltend 7, 10, 11, 12, 14, 21 oder 28 Dosiseinheiten des Gestagens.

9. Kombinationspräparat gemäß Anspruch 1 enthaltend als Gestagen Drospirenon, Dienogest oder Levonorgestrel.

10. Kombinationspräparat gemäß einem der Ansprüche 1 bis 9 zur Behandlung gynäkologischer Erkrankungen, insbesondere von uterinen Leiomyomen, der Endometriose oder von Brustkrebs.

11. Pharmazeutisches Präparat enthaltend
- einzelne Dosiseinheiten des Progesteronrezeptorantagonisten 20,20,21,21,21-Pentafluor-17-hydroxy-11β-[4-(hydroxyacetyl)phenyl]-19-nor-17α-pregna-4,9-dien-3-on der Formel 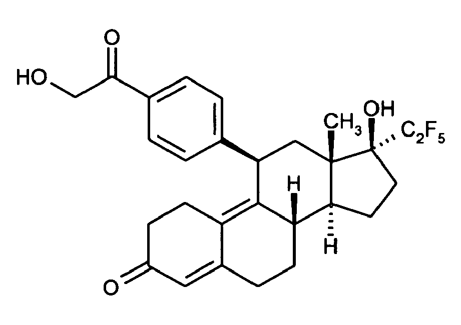 und
- eine Gebrauchsinformation, aus der hervorgeht, dass mit dem nächsten Behandlungszyklus erst mit oder nach Einsetzen der Menstruationsblutung zu beginnen ist.
